# EUROPEAN PATENT APPLICATION

(11) **EP 4 748 416 A1**
(43) Date of publication of application: **27.05.2026**
(21) Application number: 24306948.1
(22) Date of filing: 22.11.2024
(51) Int. Cl.: A61M 5/32

(54) **SYRINGE AND ASSOCIATED NEEDLE SHIELD WITH REDUCED NEEDLE PULL-OUT FORCE**

(71) Applicant: Becton Dickinson France, 38800 Le Pont-de-Claix (FR)
(72) Inventor: DORELON, Luc, 38650 Saint Martin de la Cluz (FR); OZTURK, Senturk, 38130 Echirolles (FR); VAXELAIRE, Jérémie, 74100 Ambilly (FR); DINC, Mirhac, 74960 Annecy (FR)
(74) Representative: Germain Maureau

(57) **Abstract**

Provided herein is a syringe comprising a syringe barrel including a distal barrel tip, a plunger assembly received within the syringe barrel, a needle fixedly inserted into the barrel tip, a tip ring positioned about the barrel tip and extending radially outward therefrom, and a needle shield removably connectable with the tip ring, with the needle shield positioned over the needle when connected to the tip ring. The needle shield includes a rigid outer casing defining an inner cavity, a compliant jupe positioned within the inner cavity at a distal end thereof and configured to retain the needle tip therein when the needle shield is connected to the tip ring and a compliant shield ring positioned within the inner cavity at a proximal end thereof and configured to engage the tip ring to retain the needle shield in place relative to the syringe barrel.

## Description

### BACKGROUND OF THE INVENTION

### Field of the Invention

The present disclosure relates generally to syringes and, more particularly, to syringes including a needle shield that provides container closure integrity and a reduced needle shield pull-out force.

### Description of Related Art

Syringes are used in a variety of environments for administering fluids, such as medications or other drugs, to a patient. Many syringes are provided as prefilled syringes, which provide the convenience of rapidly delivering the liquid therein to a patient without the need to first aspirate the medication from another container and meter its volume. The prefilled syringe will typically include a syringe barrel having a distal end and a proximal end. A needle is connected at the distal end for injecting fluid into the patient and a plunger assembly is inserted through the proximal end that is movable within the barrel to force fluid out from the syringe through the needle.

It is recognized that syringes must be handled with care before and after use due to the presence of the needle. To minimize the risk of accidental injury due to needle sticks, the syringe typically includes a needle shield that covers the sharpened tip of the needle. The needle shield is removed prior to use to expose the sharpened tip of the needle. Such a shield also serves to protect the sharpened tip of the needle, ensure product tightness, and preserve the sterility of the needle prior to use of the injection device.

Needle shields are generally formed to include a rigid component and a soft component or "jupe." The soft component is typically formed of elastic rubber (e.g., isoprene rubber, butyl rubber, latex rubber, silicone rubber or the like) that provides a secure sealing connection with the syringe, at least along a sealing line, and the rigid component provides protection against accidental needle sticks and provides the user with a grippable surface for the user to remove the needle shield from the syringe.

While existing needle shields and the soft component therein provide adequate protection to the needle, it is recognized that existing needle shield designs may present drawbacks with regard to usage and/or performance of the syringe.

As one example, it is desirable for the needle shield to be removable in a predictable and consistent manner. That is, with existing syringes, it is recognized that the construction of the needle shield can contribute to the "pull-out force" required to remove the needle shield from off of the syringe barrel. As examples, the formulation of the soft component of the needle shield and/or the amount and location of contact between the soft component and the (glass) syringe barrel can contribute to the amount of pull-out force required to remove the needle shield.

As another example, existing needle shields - and specifically the soft component thereof - can present issues of "coring" of the soft component into the needle. That is, it is recognized that when the needle shield is applied onto the syringe, the needle is pricked into the soft component of the needle shield to ensure a jupe-needle interface tightness and sealing. However, pricking of the needle into the soft component may cause a coring to occur, where a portion of the rubber (or other elastomeric material) is cut from the soft component and lodged within the lumen of the needle. This core of elastomeric material may thus clog the needle and negatively impact the performing of a subsequent injection using the syringe. Additionally, in some instances, the coring of the soft component by the needle can damage the beveled tip of the needle and/or alter a silicon layer applied onto the beveled tip - with any of these issues potentially impacting penetration of the needle into the patient, such as by increasing a required penetration force or increasing discomfort to a patient.

Accordingly, it is desired to provide a syringe and associated needle shield that exhibits a consistent, reduced pull-out force for removal thereof from the syringe. It is further desired that the needle shield reduces the potential for coring to occur between the needle and the soft component of the needle shield.

### SUMMARY OF THE INVENTION

Provided herein is a syringe that includes a syringe barrel having a barrel distal end and a barrel proximal end and defining a chamber, the syringe barrel including a barrel tip at the barrel distal end. The syringe also includes a plunger assembly received within the chamber of the syringe barrel and axially movable therein, a needle having a needle tip at a distal end of the needle and having a proximal end fixedly inserted into the barrel tip, and a tip ring positioned about the barrel tip and extending radially outward therefrom. The syringe further includes a needle shield removably connectable with the tip ring, with the needle shield positioned over the needle when connected to the tip ring. The needle shield comprises a rigid outer casing having a distal end portion and a proximal end portion and that defines an inner cavity. The needle shield also comprises a compliant jupe positioned within the inner cavity at the distal end portion of the rigid outer casing, the compliant jupe configured to retain the needle tip therein when the needle shield is connected to the tip ring. The needle shield further comprises a compliant shield ring positioned within the inner cavity at the proximal end portion of the rigid outer casing, the compliant shield ring configured to engage the tip ring to retain the needle shield in place relative to the syringe barrel.

In accordance with some aspects of the disclosure, the tip ring comprises a rigid tip ring.

In accordance with some aspects of the disclosure, the tip ring comprises a conical ring having a distal-facing surface formed as an angled surface.

In accordance with some aspects of the disclosure, the tip ring comprises one or more annular rings formed circumferentially thereabout and configured to engage the compliant shield ring.

In accordance with some aspects of the disclosure, the compliant jupe and the compliant shield ring are formed as separate components and are separated from one another within the inner cavity in a lengthwise direction of the needle shield.

In accordance with some aspects of the disclosure, the inner cavity comprises a tip ring storage portion extending substantially between the proximal end portion and the distal end portion of the rigid outer casing and configured to receive and retain the compliant jupe therein, and a jupe storage portion positioned proximally from the jupe storage portion at the proximal end portion of the rigid outer casing, the jupe storage portion forming a seat configured to receive and retain the shield ring therein.

In accordance with some aspects of the disclosure, the tip ring storage portion has an inner diameter that is greater than an inner diameter of the jupe storage portion, such that the inner cavity has a stepped configuration.

In accordance with some aspects of the disclosure, the tip ring defines an opening therein, the opening having an inner diameter less than an outer diameter of the barrel tip, such that the tip ring is secured on the barrel tip via an interference fit.

In accordance with some aspects of the disclosure.

In accordance with some aspects of the disclosure, each of the compliant jupe and the compliant shield ring is formed of a compliant material that comprises an elastic rubber or a thermoplastic elastomer.

In accordance with some aspects of the disclosure, the compliant jupe is formed of a first compliant material and the compliant shield ring is formed of a second compliant material different from the first compliant material.

In accordance with some aspects of the disclosure, a hardness of the first compliant material of the compliant jupe comprises a Shore A hardness of 45 or less, and wherein a hardness of the second compliant material of the compliant shield ring comprises a Shore A hardness of 60 or more.

In accordance with some aspects of the disclosure, the compliant shield ring engages the tip ring such that a pull-out force of between 5N and 25N is required to remove the needle shield from the tip ring.

Also provided herein is a needle shield for use with a syringe comprising a syringe barrel having a distal barrel tip and a needle secured in the distal barrel tip. The needle shield includes a rigid outer casing having a distal end portion and a proximal end portion, the rigid outer casing defining an inner cavity. The needle shield also includes a compliant jupe positioned within the inner cavity at the distal end portion of the rigid outer casing, the compliant jupe configured to retain a needle tip of the needle therein when the needle shield is connected to the syringe barrel. The needle shield further includes a compliant shield ring positioned within the inner cavity at the proximal end portion of the rigid outer casing, the compliant shield ring configured to retain the needle shield in place relative to the syringe barrel.

In accordance with some aspects of the disclosure, the compliant jupe and the compliant shield ring are formed as separate components and are separated from one another within the inner cavity in a lengthwise direction of the needle shield.

In accordance with some aspects of the disclosure, the inner cavity comprises a tip ring storage portion extending substantially between the proximal end portion and the distal end portion of the rigid outer casing and configured to receive and retain the compliant jupe therein, and a jupe storage portion positioned proximally from the jupe storage portion at the proximal end portion of the rigid outer casing, the jupe storage portion forming a seat configured to receive and retain the shield ring therein.

In accordance with some aspects of the disclosure, the tip ring storage portion has an inner diameter that is greater than an inner diameter of the jupe storage portion, such that the inner cavity has a stepped configuration.

In accordance with some aspects of the disclosure, each of the compliant jupe and the compliant shield ring is formed of a compliant material that comprises an elastic rubber or a thermoplastic elastomer.

In accordance with some aspects of the disclosure, the compliant jupe is formed of a first compliant material and the compliant shield ring is formed of a second compliant material different from the first compliant material, where the first compliant material has a Shore A hardness that is less than a Shore A hardness of the second compliant material.

In accordance with some aspects of the disclosure, the compliant shield ring is configured to engage a rigid tip ring positioned about the distal barrel tip of the syringe barrel.

In accordance with some aspects of the disclosure, the compliant shield ring engages the tip ring such that a pull-out force of between 5N to 25N is required to remove the needle shield from the tip ring.

### BRIEF DESCRIPTION OF THE DRAWINGS

FIG. 1 is a perspective view of a syringe including a needle shield, according to an embodiment of the disclosure;
FIG. 2 is an exploded view of the syringe of FIG. 1;
FIG. 3A is a cross-sectional view of the needle shield of FIG. 1 taken along line A-A, according to an embodiment of the disclosure embodiment, with the needle shield attached to the syringe barrel;
FIG. 3B shows the needle shield of FIG. 3A with the needle shield detached from the syringe barrel;
FIG. 3C shows the needle shield of FIG. 3A with the needle shield detached from the syringe barrel;
FIG. 4A is a cross-sectional view of the needle shield of FIG. 1 taken along line A-A, according to another embodiment of the disclosure embodiment, with the needle shield attached to the syringe barrel;
FIG. 4B shows the needle shield of FIG. 4A with the needle shield detached from the syringe barrel; and
FIG. 4C shows the needle shield of FIG. 4A with the needle shield detached from the syringe barrel.

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

In the present disclosure, the distal end of a component or of a device means the end furthest away from the hand of the user and the proximal end means the end closest to the hand of the user, when the component or device is in the use position, i.e., when the user is holding a syringe in preparation for or during use. Similarly, in this application, the terms "in the distal direction" and "distally" mean in the direction toward the distal tip of the syringe, and the terms "in the proximal direction" and "proximally" mean in the direction opposite the direction of the distal tip of the syringe.

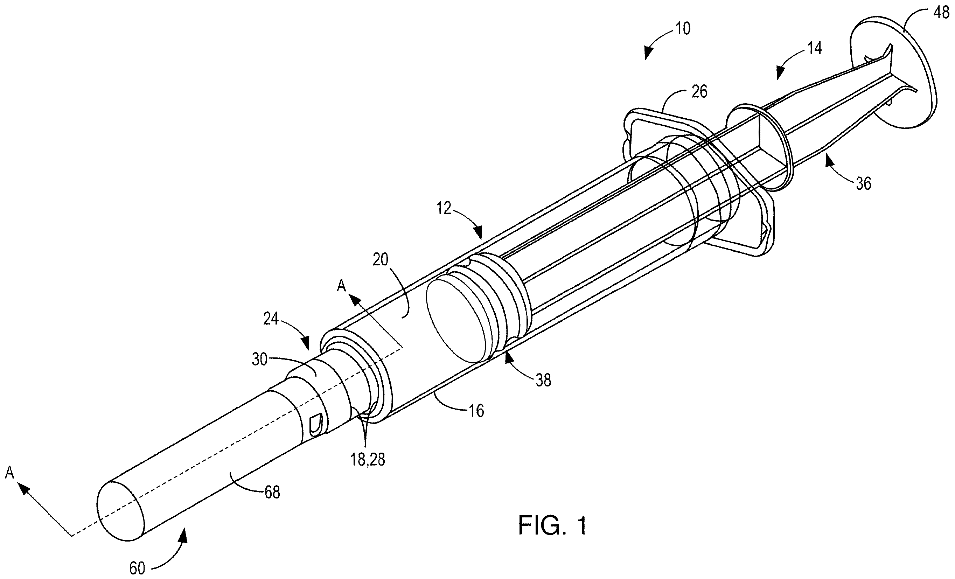

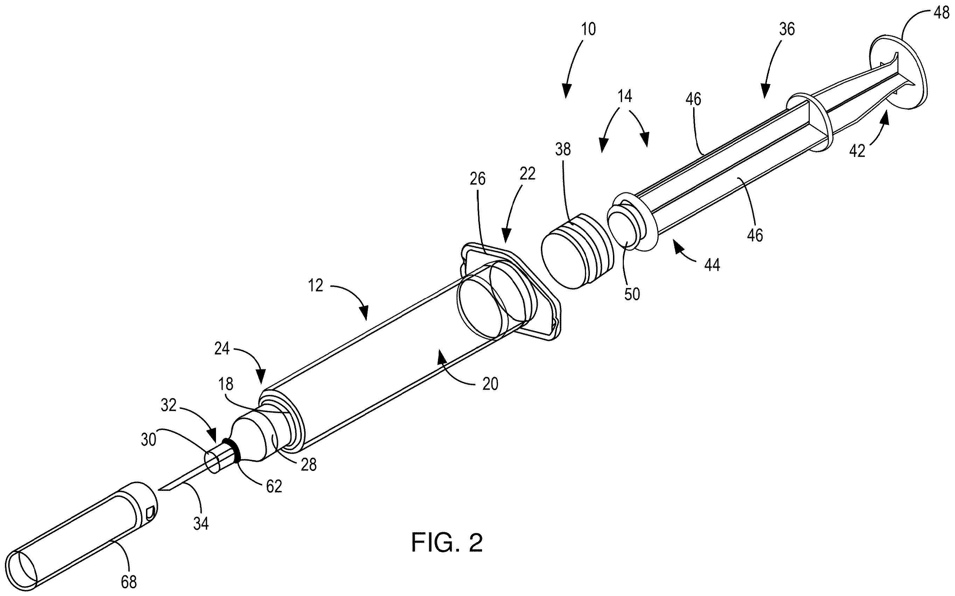

Referring to FIGS. 1 and 2, shown is a non-limiting embodiment of a medical injection device 10, such as a syringe for example, with which aspects or embodiments of the disclosure may be implemented. While the medical injection device is shown and described hereafter as a syringe 10, it is recognized that other medical injection devices may incorporate aspects of the disclosure, as described in detail here below.

As shown in FIGS. 1 and 2, the syringe 10 generally includes a syringe barrel 12 and a plunger assembly 14. The plunger assembly 14 is movable within the syringe barrel 12 along a longitudinal axis to an advanced position to facilitate administering of an injectable fluid (e.g., medication) to a patient, for example. The syringe barrel 12 is formed of a generally cylindrical outer wall 16 and an end member 18 that collectively define a chamber 20 for retaining fluid therein. The syringe barrel 12 includes an open proximal end 22 configured to receive the plunger assembly 14 therein and a distal end 24 at which end member 18 is positioned. The proximal end 22 of the syringe barrel 12 may include a flange 26 to facilitate handling and positioning of the syringe 10 and to maintain the relative position of the syringe barrel 12 with respect to the plunger assembly 14 during medication administration. At the distal end 24, the end member 18 may include a shoulder 28 which narrows with respect to the cylindrical outer wall 16, as well as a hub portion or tip 30 extending out distally from shoulder 28. The tip 30 is formed as a partially hollow member that defines a channel 32 therethrough in fluid communication with the chamber 20. A needle 34 is attached to the tip 30 within channel 32, with a proximal end of the needle 34 positioned within the channel 32 and a distal end of the needle 34 extending out from the tip 30. The proximal end of the needle 34 may be inserted into the channel 32 of tip 30 and may be fixed therein with an adhesive (e.g., glue), by thermal welding, or the like. The needle 34 defines a hollow lumen therein that is in fluid communication with the chamber 20 of syringe barrel 12, such that fluid may be dispensed from the syringe 10.

The plunger assembly 14 of syringe 10 is formed of an elongate plunger rod 36 (more generally "plunger 36," as used hereafter) and a plunger head or stopper 38. The plunger 36 may include a main body 40 extending between a plunger proximal end 42 and a plunger distal end 44. In some embodiments, the main body 40 may include a plurality of elongate vanes or walls 46 extending axially along a length thereof between the plunger proximal end 42 and the plunger distal end 44. A thumb press 48 is positioned at the plunger proximal end 42 that may be engaged by a thumb (or other finger) of the user to apply a distally directed force to the plunger assembly 14 to move the plunger 36 with respect to the syringe barrel 12. In some embodiments, a flanged extension member 50 (e.g., disc-shaped flange) is positioned at the plunger distal end 44 that is configured to mate with the stopper 38. In other embodiments, the plunger distal end 44 may include a female receptacle formed therein that is configured to receive and connect to a protrusion (e.g., pin) extending out proximally from the stopper, with the protrusion and receptacle engaging via threading, for example.

The stopper 38 of plunger assembly 14 is positioned at the plunger distal end 44 so as to be movable along with the plunger 36 within the chamber 20 of syringe barrel 12. The stopper 38 may be made from a material that is different from the material of the plunger 36 and that is capable of forming a tight seal with the syringe barrel 12 as it is advanced therethrough. In some embodiments, the stopper 38 includes a receptacle (not shown) formed therein that is sized and configured to receive the flanged extension member 50 of plunger 36, with the flanged extension member 50 coupling with the receptacle via a press fit connection, for example, to secure the stopper 38 to the plunger 36, although it can be appreciated that the stopper 38 and plunger distal end 44 can be secured by other techniques known in the art.

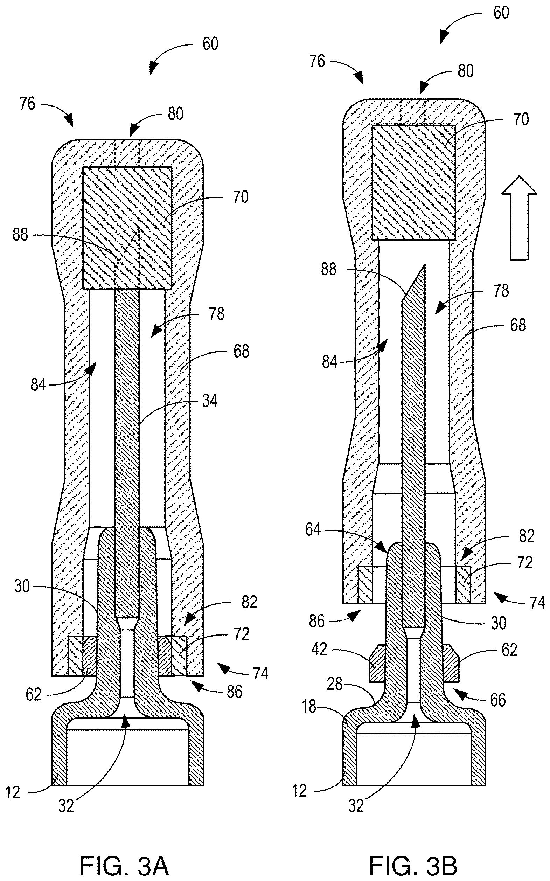

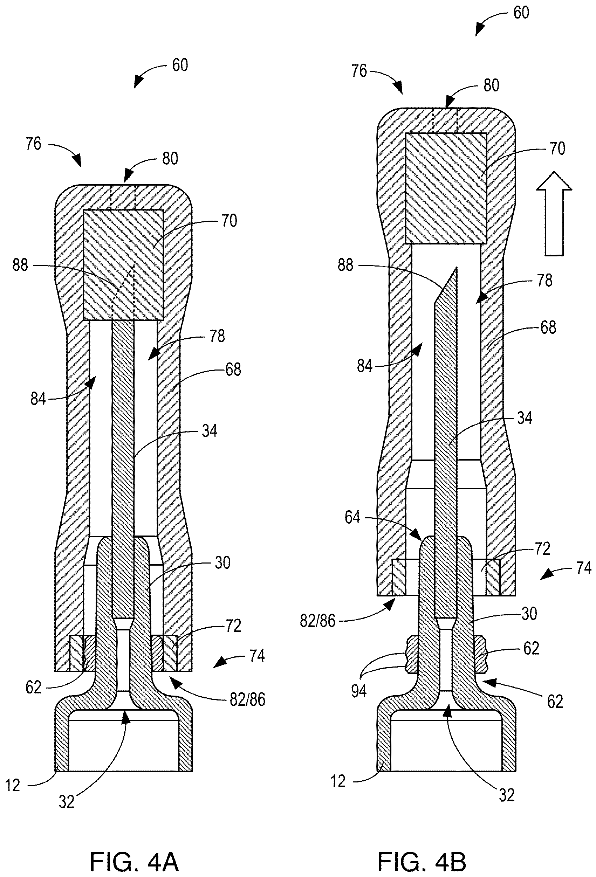

As shown in FIGS. 1 and 2, a needle shield 60 of syringe assembly 12 is coupled to the syringe barrel 12 to protect the needle 34. According to aspects of the disclosure, the needle shield 60 is configured to be mounted to the barrel 12 via coupling of the needle shield 60 to the tip 30 of the barrel 16. More specifically, the needle shield 60 may be coupled to the tip 30 of the barrel 16 via mating of the needle shield 60 with a tip ring 62 positioned on/about the tip 30, as best shown in FIG. 2. Th tip ring 62 has an annular shape and is positioned on tip 30 so as to extend radially outward therefrom. The tip ring 62 may comprise a rigid component, such as a molded polymeric component, that is positioned about the tip 30 at a location between a distal end 64 and a proximal end 66 thereof (e.g., near a proximal end 66 of the tip 30, see FIGS. 3A-3C and FIGS. 4A-4C) - with the tip ring 62 secured on the tip 30 via a friction or interference fit, as non-limiting examples. With a tight/high interference between the tip ring 62 and the tip 30 of syringe barrel 12, container closure integrity can be ensured upon connection of the needle shield 60 to the syringe barrel 12 (e.g., a resistance to dye test may be performed on the proximal side of the needle shield 60 to ensure container closure integrity).

As explained in further detail below, the needle shield 60 may be composed of a rigid material or semi-rigid material that provides structure to the needle shield 60, as well as one or more elastomeric materials (i.e., a deformable or compliant materials) that surround the needle 34 when the needle shield 60 is coupled to the syringe barrel 12 and that present a mating surface for the tip 30 of barrel 12. In various embodiments, the rigid material may be a resin such as polypropylene (PP), polyethylene (PE), polyethylene terephthalate (PET), polystyrene (PS), polycarbonate (PC), or polyvinyl chloride (PVC), as non-limiting examples, while the elastomeric material(s) may be an elastic rubber (e.g., isoprene rubber, butyl rubber, latex rubber, silicone rubber or the like) or a thermoplastic elastomer (TPE), as non-limiting examples.

Referring now to FIGS. 3A-3C and FIGS. 4A-4C, the needle shield 60 is shown in further detail in accordance with an aspect of the disclosure. As shown therein, the needle shield 60 is composed of a rigid outer casing 68 that provides structure to the needle shield 60, a compliant jupe 70 positioned within the rigid outer casing 68 that surrounds the needle 34 (when needle shield 60 is positioned on syringe barrel 12), and a compliant shield ring 72 positioned within the rigid outer casing 68 that mates with the tip ring 62 (when needle shield 60 is positioned on syringe barrel 12).

The rigid outer casing 68 may be constructed as a generally tubular member that includes an open proximal end portion 74 and a mostly closed distal end portion 76, with the rigid outer casing defining an inner cavity 78 therein. The open proximal end portion 74 allows for positioning of the needle shield 60 onto the tip 30 of syringe barrel 12, with the tip 30 and needle 34 being introduced through the open proximal end 74 into the inner cavity 78. In some embodiments, the distal end portion 76 may include an opening 80 therein having a smaller diameter than a diameter of the compliant jupe 70, such that the compliant jupe 70 may be better retained in the rigid outer casing 68.

According to aspects of the disclosure, the inner cavity 76 may be characterized as including a tip ring storage portion 82 and a jupe storage portion 84.

The jupe storage portion 84 of inner cavity 76 extends substantially between the proximal end portion 74 and the distal end portion 76 of the rigid outer casing 68. The jupe storage portion 84 is sized to receive the compliant jupe 70 therein, with an inner diameter of the jupe storage portion 84 approximately equal to (or slightly smaller than) an outer diameter of the compliant jupe 70, such that the compliant jupe 70 may be securely retained within the jupe storage portion 84.

The tip ring storage portion 82 of inner cavity 76 is positioned proximally from the jupe storage portion 84 and at the open proximal end portion 74 of the rigid outer casing 68. As can be seen in FIGS. 3A-3C and FIGS. 4A-4C, the tip ring storage portion 82 has an inner diameter that is greater than the inner diameter of the jupe storage portion 84, such that the inner cavity 76 may be considered to have a stepped configuration. With this stepped configuration of the inner cavity 76, the tip ring storage portion 82 may thus provide a seat 86 within which the compliant shield ring 72 may be positioned and retained.

According to embodiments, the compliant jupe 70 is configured to be positioned within the jupe storage portion 84 of inner cavity 76, at a distal end thereof, such that the compliant jupe 70 is positioned at the distal end portion 76 of the rigid outer casing 68. The compliant jupe 70 is positioned such that a beveled needle tip 88 of needle 34 punctures and extends into the compliant jupe 70 once the needle shield 60 is coupled to barrel 12, with the needle tip 88 thus being sealed in the compliant jupe 70. With the needle tip 88 sealed in the compliant jupe 70, leakage of fluid out from the needle 34 may be prevented. As previously indicated, an outer diameter of the compliant jupe 70 may be approximately equal to (or slightly greater than) an inner diameter of the jupe storage portion 84, such that an interference is provided between the compliant jupe 70 and the rigid outer casing 68 that securely retains the compliant jupe 70 in-place therein.

The compliant shield ring 72 is configured to be positioned within the tip ring storage portion 82 of inner cavity 76. The compliant shield ring 72 has an outer diameter that is slightly greater than an inner diameter of the tip ring storage portion 82, such that an interference is provided between the compliant shield ring 72 and the rigid outer casing 68 that securely retains the compliant shield ring 72 in-place in the tip ring storage portion 82. The compliant shield ring 72 has an annular shape that defines an opening 90 therein. The opening 90 receives the tip ring 62 therein when the needle shield 60 is positioned on the syringe barrel 12, with the tip ring 62 brought into contact with the compliant shield ring 72. According to embodiments, the opening 90 is sized such that a radial interference is present between the rigid tip ring 62 and compliant shield ring 72, such that the needle shield 60 may be securely retained on syringe barrel 12. According to embodiments, the mating of the rigid tip ring 62 and compliant shield ring 72 is configured to provide a consistent pull-out force needed to remove the needle shield 60 from the syringe barrel 12. In some embodiments, a pull-out force of between 5N to 25N may be sufficient to remove the needle shield 60 from the syringe barrel 12. This pull-out force may be compared to a pull-out force of up to 45N that may be required to remove a needle shield with a known design/configuration from a syringe barrel, where a compliant member of the needle shield is in direct contact with the glass tip of the syringe barrel.

As can be send in FIGS. 3A-3C and FIGS. 4A-4C, the compliant jupe 70 and the compliant shield ring 72 are formed as separate components (e.g., such as via a bi-injection process) and are separated from each other within the cavity 76 in a lengthwise direction of the needle shield 60. According to some aspects of the disclosure, it is recognized that it may be desirable to form the compliant jupe 70 and the compliant shield ring 72 from a combination of different elastomeric materials. That is, in some instances, it may be desirable to form the compliant jupe 70 from a softer elastomeric material that is more resistant to coring, while the compliant shield ring 72 is formed from a stiffer elastomeric material that is durable and provides a desired for an interface with the rigid tip ring 62 (to promote securing/sealing therebetween).

According to aspects of the disclosure, the compliant jupe 70 may be formed of a first material having a shore hardness that is lower than the shore hardness of a second material from which compliant shield ring 72 is formed - i.e., the first material is softer than the second material. The lower shore hardness of the first material of compliant jupe 70 - into which the tip 88 of needle 34 punctures and extends - reduces the potential for (1) coring or chipping of the material to occur when the needle is inserted into the first material, and/or (2) damaging of the beveled needle tip 88 when the needle 34 is inserted into the first material, as the softer first material is more able to deform and "give" when the needle 34 is inserted therein. The higher shore hardness of the second material of compliant shield ring 72 helps in forming a seal with the rigid tip ring 62 provided on the tip 30 of the syringe barrel 12.

According to some embodiments, the first material and the second material may be TPE resins of differing hardness, as a non-limiting example. In some non-limiting embodiments, the first material preferably has a Shore A hardness of 45 of less, while the second material preferably has a Shore A hardness of 60 or more. With these hardness values, the compliant jupe 70 and compliant shield ring 72 may provide the functionality as described above.

Referring now to the embodiments of FIGS. 3A-3C and FIGS. 4A-4C, specific configurations of the rigid tip ring 62 are provided with which the compliant shield ring 72 can mate in order to provide a desired needle shield pull-out force.

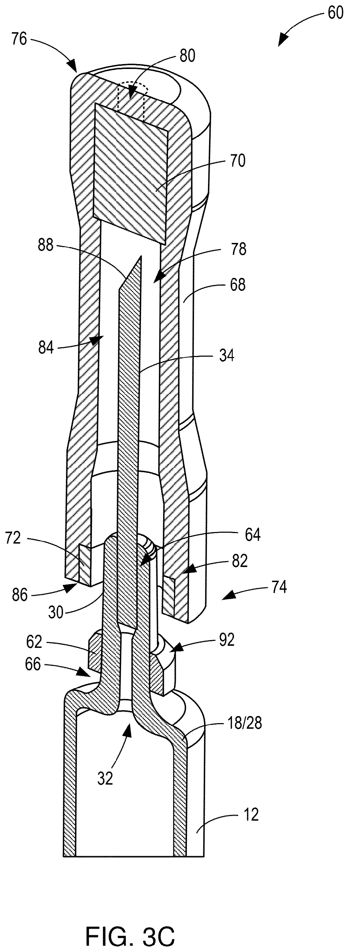

Referring first to FIGS. 3A-3C, an embodiment is illustrated where rigid tip ring 62 is constructed as a conical washer or ring, where a distal-facing surface of the tip ring 62 is formed as an angled surface 92. The angled distal-facing surface 92 of the tip ring 62 is configured to engage the compliant shield ring 72 when the needle shield 60 is pushed onto the syringe barrel 12, with the angled distal-facing surface 92 providing a secure engagement with the compliant shield ring 72 and ensuring container closure integrity for the syringe 10. FIG. 3A illustrates the needle shield 60 positioned on the syringe barrel 12, with engagement between the tip ring 62 and the compliant shield ring 72 keeping the needle shield 60 securely in place. FIGS. 3B and 3C show the needle shield 60 being removed from the syringe barrel 12 responsive to a distally-directed pulling force being applied to the needle shield 60. As previously described, the engagement between the rigid (plastic) tip ring 62 and the compliant shield ring 72 provides a consistent, reduced pull-out force that is required to remove the needle shield 60, as compared to higher and unpredictable pull-out force that may be needed to remove a needle shield where a unitary compliant jupe structure in the needle shield directly contacts the glass material of a syringe barrel.

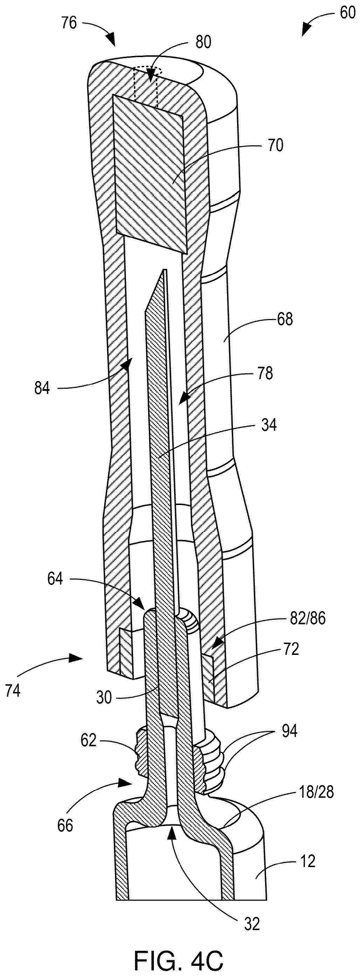

Referring next to FIGS. 4A-4C, an embodiment is illustrated where rigid tip ring 62 is constructed as a ribbed tip ring, where one or more annular rings (i.e., O-rings) 94 are formed circumferentially about the tip ring 62. The annular rings 94 are configured to engage the compliant shield ring 72 when the needle shield 60 is pushed onto the syringe barrel 12, with the annular rings 94 providing a secure engagement with the compliant shield ring 72 and ensuring container closure integrity for the syringe 10. FIG. 4A illustrates the needle shield 60 positioned on the syringe barrel 12, with engagement between the tip ring 62 and the compliant shield ring 72 keeping the needle shield 60 securely in place. FIGS. 4B and 4C show the needle shield 60 being removed from the syringe barrel 12 responsive to a distally-directed pulling force being applied to the needle shield 60. As previously described, the engagement between the rigid (plastic) tip ring 62 and the compliant shield ring 72 provides a consistent, reduced pull-out force that is required to remove the needle shield 60, as compared to higher and unpredictable pull-out force that may be needed to remove a needle shield where a unitary compliant jupe structure in the needle shield directly contacts the glass material of a syringe barrel.

Beneficially, embodiments of the disclosure provide a syringe and associated needle shield that exhibits a consistent, reduced pull-out force for removal thereof from the syringe. The needle shield includes a compliant needle jupe and compliant shield ring contained within a rigid outer housing. The compliant shield ring mates with a rigid tip ring provided on a tip of the syringe barrel to provide for engagement between the needle shield and the syringe barrel, with the mating between the compliant shield ring and rigid tip ring allowing for a consistent, reduced pull-out force when removing the needle shield from the syringe. The compliant needle jupe and compliant shield ring are provided as separate structures, such that needle retention within the compliant needle jupe is independent from the pull-out force required to disengage the compliant shield ring from the rigid tip ring. The separate compliant needle jupe and compliant shield ring also allow for forming of these components from different compliant materials, thus allowing the compliant needle jupe to be formed specifically to reduce the potential for coring to occur between the needle and the needle jupe. The separate compliant needle jupe and compliant shield ring also reduces the amount of compliant material contained in the needle shield as compared to conventional needle shields, so as to reduce material costs. Still further, container closure integrity can be ensured by a high level of interference between the rigid tip ring and the syringe tip.

Although the present disclosure has been described in detail for the purpose of illustration based on what is currently considered to be the most practical and preferred embodiments or aspects, it is to be understood that such detail is solely for that purpose and that the present disclosure is not limited to the disclosed embodiments or aspects, but, on the contrary, is intended to cover modifications and equivalent arrangements that are within the spirit and scope of the appended claims. For example, it is to be understood that the present disclosure contemplates that, to the extent possible, one or more features of any embodiment may be combined with one or more features of any other embodiment.

## Claims

1. A syringe (10) comprising:
a syringe barrel (12) having a barrel distal end and a barrel proximal end and defining a chamber, the syringe barrel including a barrel tip at the barrel distal end;
a plunger assembly (14) received within the chamber of the syringe barrel and axially movable therein;
a needle (34) having a needle tip at a distal end of the needle and having a proximal end fixedly inserted into the barrel tip;
a tip ring (62) positioned about the barrel tip and extending radially outward therefrom; and
a needle shield (60) removably connectable with the tip ring, with the needle shield positioned over the needle when connected to the tip ring, wherein the needle shield comprises:
a rigid outer casing (68) having a distal end portion and a proximal end portion, the rigid outer casing defining an inner cavity;
a compliant jupe (70) positioned within the inner cavity at the distal end portion of the rigid outer casing, the compliant jupe configured to retain the needle tip therein when the needle shield is connected to the tip ring; and
a compliant shield ring (72) positioned within the inner cavity at the proximal end portion of the rigid outer casing, the compliant shield ring configured to engage the tip ring to retain the needle shield in place relative to the syringe barrel.

2. The syringe (10) of claim 1, wherein the tip ring (62) comprises a rigid tip ring.

3. The syringe (10) of any of claims 1-2, wherein the tip ring (62) comprises a conical ring having a distal-facing surface formed as an angled surface.

4. The syringe (10) of any of claims 1-3, wherein the tip ring (62) comprises one or more annular rings formed circumferentially thereabout and configured to engage the compliant shield ring (72).

5. The syringe (10) of any of claims 1-4, wherein the compliant jupe (70) and the compliant shield ring (72) are formed as separate components and are separated from one another within the inner cavity in a lengthwise direction of the needle shield (60).

6. The syringe (10) of any of claims 1-5, wherein the inner cavity comprises:
a tip ring storage portion (82) extending substantially between the proximal end portion and the distal end portion of the rigid outer casing (68) and configured to receive and retain the compliant jupe (70) therein; and
a jupe storage portion (84) positioned proximally from the jupe storage portion at the proximal end portion of the rigid outer casing, the jupe storage portion forming a seat configured to receive and retain the shield ring (72) therein;
wherein, optionally, the tip ring storage portion has an inner diameter that is greater than an inner diameter of the jupe storage portion, such that the inner cavity has a stepped configuration.

7. The syringe (10) of any of claims 1-6, wherein the tip ring (62) defines an opening therein, the opening having an inner diameter less than an outer diameter of the barrel tip, such that the tip ring is secured on the barrel tip via an interference fit.

8. The syringe (10) of any of claims 1-7, wherein each of the compliant jupe (70) and the compliant shield ring (72) is formed of a compliant material that comprises an elastic rubber or a thermoplastic elastomer;
wherein, optionally, the compliant jupe is formed of a first compliant material and the compliant shield ring is formed of a second compliant material different from the first compliant material; and
wherein, optionally, a hardness of the first compliant material of the compliant jupe comprises a Shore A hardness of 45 or less, and wherein a hardness of the second compliant material of the compliant shield ring comprises a Shore A hardness of 60 or more.

9. The syringe (10) of any of claims 1-8, wherein the compliant shield ring (72) engages the tip ring (62) such that a pull-out force of between 5N to 25N is required to remove the needle shield (60) from the tip ring.

10. A needle shield (60) for use with a syringe (10) comprising a syringe barrel (12) having a distal barrel tip and a needle (34) secured in the distal barrel tip, the needle shield comprising:
a rigid outer casing (68) having a distal end portion and a proximal end portion, the rigid outer casing defining an inner cavity;
a compliant jupe (70) positioned within the inner cavity at the distal end portion of the rigid outer casing, the compliant jupe configured to retain a needle tip of the needle therein when the needle shield is connected to the syringe barrel; and
a compliant shield ring (72) positioned within the inner cavity at the proximal end portion of the rigid outer casing, the compliant shield ring configured to retain the needle shield in place relative to the syringe barrel.

11. The needle shield (60) of claim 10, wherein the compliant jupe (70) and the compliant shield ring (72) are formed as separate components and are separated from one another within the inner cavity in a lengthwise direction of the needle shield.

12. The needle shield (60) of any of claims 10-11, wherein the inner cavity comprises:
a tip ring storage portion (82) extending substantially between the proximal end portion and the distal end portion of the rigid outer casing (68) and configured to receive and retain the compliant jupe (70) therein; and
a jupe storage portion (84) positioned proximally from the jupe storage portion at the proximal end portion of the rigid outer casing, the jupe storage portion forming a seat configured to receive and retain the shield ring (72) therein.

13. The needle shield (60) of claim 12, wherein the tip ring storage portion (82) has an inner diameter that is greater than an inner diameter of the jupe storage portion (84), such that the inner cavity has a stepped configuration.

14. The needle shield (60) of any of claims 10-13, wherein each of the compliant jupe (70) and the compliant shield ring (72) is formed of a compliant material that comprises an elastic rubber or a thermoplastic elastomer; and
wherein, optionally, the compliant jupe is formed of a first compliant material and the compliant shield ring is formed of a second compliant material different from the first compliant material, where the first compliant material has a Shore A hardness that is less than a Shore A hardness of the second compliant material.

15. The needle shield (60) of any of claims 10-14, wherein the compliant shield ring (72) is configured to engage a rigid tip ring (62) positioned about the distal barrel tip of the syringe barrel (12); and
wherein, optionally, the compliant shield ring engages the tip ring such that a pull-out force of between 5N to 25N is required to remove the needle shield from the tip ring.
